# DEMANDE DE BREVET EUROPEEN

(11) **EP 0 891 773 A1**
(43) Date de publication de la demande: **20.01.1999**
(21) Numéro de dépôt: 98401370.6
(22) Date de dépôt: 08.06.1998
(51) Int. Cl.: A61K 31/20, A61K 7/48

(54) **Utilisation de l'acide gamma-linolenique pour prévenir le stress oxydatif**

(30) Priorité: 07.07.1997 FR 9708607
(71) Demandeur: L'OREAL, 75008 Paris (FR)
(72) Inventeur: Galey, Isabelle, 78000 Versailles (FR); Duranton, Albert, 75018 Paris (FR)
(74) Mandataire: Tezier Herman, Béatrice

(57) **Abrégé**

L'invention a pour objet l'utilisation d'acide γ-linolénique à titre de principe actif dans une composition cosmétique ce composé ou la composition étant destiné à prévenir les effets du stress oxydatif sur la peau et/ou le vieillissement cutané.

## Description

La présente invention a pour objet l'utilisation à titre de principe actif dans une composition cosmétique de l'acide γ-linolénique, ce composé ou la composition étant destiné à prévenir les effets du stress oxydatif sur la peau.

Particulièrement, l'invention a pour objet l'utilisation à titre de principe actif dans une composition cosmétique de l'acide γ-linolénique, ce composé ou la composition étant destiné à prévenir le vieillissement cutané.

Le rôle de l'oxydation cellulaire dans le vieillissement cutané, qu'il soit intrinsèque ou extrinsèque, notamment photo-induit, est connu. Ce vieillissement cutané se traduit par différents signes cliniques notamment l'apparition de ridules et de rides profondes, en augmentation avec l'âge. Par ailleurs, l'apparence de la peau ou du scalp se détériore. Le teint de la peau est généralement modifié et il peut exister sur certaines zones des irritations diffuses. Un autre signe clinique du vieillissement est l'aspect sec et rêche de la peau qui est dû essentiellement à une desquamation plus importante. On constate enfin une perte de fermeté et de tonicité de la peau qui, comme pour les rides et les ridules, s'explique du moins en partie par une atrophie dermique et épidermique ainsi qu'un aplatissement de l'ensemble derme/épiderme.
Ainsi, les signes cliniques du vieillissement cutané résultent essentiellement d'un dysfonctionnement des principaux mécanismes biologiques intervenant au niveau de la peau. Parmi ces mécanismes biologiques, le stress oxydatif contribue largement au phénomène de vieillissement cutané.

Le stress oxydatif est le résultat d'un déséquilibre entre d'une part la production d'espèces d'oxygène réactives, incluant l'anion superoxyde, le peroxyde d'hydrogène et le radical hydroxyle, et d'autre part les mécanismes antioxydants de défense cellulaire. Les espèces oxygène réactives sont produites durant la respiration, l'inflammation et d'autres événements métaboliques cellulaires.
Le stress oxydatif est également lié à des agents extérieurs comme les rayonnements ultraviolets, la pollution ou encore divers agents chimiques avec lesquels la peau peut être en contact (chlore, produits de nettoyage, etc.).
La production d'espèces d'oxygène réactives provoque des dommages au niveau de l'ADN, des protéines ou des lipides. Les cellules ont développé des mécanismes élaborés pour échapper à ces conséquences dramatiques comme par exemple des enzymes (superoxyde dismutase, catalase, glutathion peroxydase) ou encore des systèmes antioxydants non-enzymatiques.

Il n'en demeure pas moins que le désir de conserver une apparence jeune conduit à la recherche incessante de compositions permettant de maintenir ou d'améliorer l'apparence de la peau.

De manière surprenant et inattendue, la demanderesse a maintenant découvert que l'acide γ-linolénique peut constituer un excellent actif pour prévenir le stress oxydatif.

Il a déjà été proposé des compositions associant divers ingrédients à de l'acide γ-linolénique pour combattre le vieillissement cutané.
Par exemple dans le brevet suédois n°8802243-9, il est proposé d'associer l'acide γ-linolénique à du trinitrate de glycérol. Ce dernier composé, décrit comme le composé le plus important de la composition, permet l'oxydation des espèces d'oxygène réactives, les empêchant ainsi de dégrader les acides gras polyinsaturés des cellules de la peau. La composition apporte l'acide γ-linolénique pour remplacer les acides gras polyinsaturés déjà dégradés par les espèces d'oxygène réactives.
Dans la demande de brevet européen n°279 136, il est proposé une composition cosmétique pour retarder le vieillissement de la peau comprenant une première composition comportant un actif hydrosoluble et une deuxième composition comportant un principe actif liposoluble. La composition peut en outre contenir des composés sans influence directe sur le processus de vieillissement par les radicaux libres comme par exemple de l'acide γ-linolénique.

Ainsi, à la connaissance de la demanderesse il n'a jamais été proposé d'utiliser l'acide γ-linolénique comme actif pour prévenir les effets des radicaux libres.

Comme il est montré dans les exemples, l'acide γ-linolénique a la propriété de protéger les cellules de la peau du stress oxydatif. Ainsi, il est possible par une application préalable d'une composition comprenant une quantité efficace d'acide γ-linolénique de prévenir les effets des radicaux libres.

Avantageusement, l'invention a pour objet l'utilisation à titre de principe actif dans une composition cosmétique d'une quantité efficace d'acide γ-linolénique, ce composé ou la composition étant destiné à prévenir les effets du stress oxydatif sur la peau.

L'invention a plus particulièrement pour objet l'utilisation à titre de principe actif dans une composition cosmétique d'une quantité efficace d'acide γ-linolénique, ce composé ou la composition étant destiné à prévenir le vieillissement cutané.

L'invention a enfin pour objet une composition cosmétique comprenant dans un milieu physiologiquement acceptable de l'acide γ-linolénique à titre d'actif destiné à prévenir les effets du stress oxydatif ou destiné à prévenir le vieillissement cutané.

La quantité d'acide γ-linolénique contenue dans les compositions de l'invention est bien entendu fonction de l'effet recherché et peut donc varier dans une large mesure.

Pour donner un ordre de grandeur, dans les compositions utilisées selon l'invention l'acide γ-linolénique est en une proportion comprise entre 0,05% et 10% et préférentiellement comprise entre 1% et 5% en poids par rapport au poids de la composition.

Les compositions utilisables selon l'invention peuvent se présenter sous toutes les formes galéniques normalement utilisées. Préférentiellement, ces compositions sont destinées à être appliquées sur la peau.

Pour une application topique sur la peau, la composition peut avoir la forme notamment d'une solution aqueuse, alcoolique, hydroalcoolique ou huileuse ou d'une dispersion du type lotion ou sérum, d'une émulsion de consistance liquide ou semi-liquide du type lait, obtenue par dispersion d'une phase grasse dans une phase aqueuse (H/E) ou inversement (E/H), ou d'une suspension ou d'une émulsion de consistance molle du type crème, mousse ou gel aqueux ou anhydre, ou encore de microcapsules ou microparticules, ou d'une dispersion vésiculaire de type ionique et/ou non ionique. Elle peut également prendre la forme d'une composition pour aérosol comprenant également un agent propulseur sous pression.

La composition est généralement appliquée sur la partie (peau ou muqueuses) à traiter, puis peut être éventuellement maintenue en position par une pièce (patch) adhésive. La pièce adhésive peut permettre une occlusion partielle ou totale de la composition sur la partie à traiter. On peut par exemple utiliser, comme pansement adhésif, le produit Tegaderm® vendu par les Laboratoires 3M Santé ou, comme pièce adhésive, le produit Finn Chambers® vendu par la société Promédica.

Quelle que soit sa forme, cette composition est préparée selon les méthodes usuelles.

Les quantités des différents constituants de la composition selon l'invention sont celles classiquement utilisées dans les domaines considérés.

Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

On peut également envisager que la composition soit sous forme liposomée, telle que notamment décrite dans la demande de brevet WO 94/22468 déposée le 13 octobre 1994 par la société Anti Cancer Inc.

Cette composition peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).

Comme émulsionnants utilisables dans l'invention, on peut citer par exemple le stéarate de glycérol, le polysorbate 60 et le mélange PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Selon l'invention on peut, entre autres, associer l'acide γ-linolénique à d'autres agents actifs destinés notamment à la prévention et/ou au traitement des affections cutanées. Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides alpha- et bêta- hydroxycarboxyliques ou bêta-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle.
- des substances telles que les antagonistes de substance P, de CGRP ou de bradykinine ou les inhibiteurs de NO synthase, composés décrits comme étant actifs dans le traitement des peaux sensibles et comme présentant des effets anti-irritant, en particulier vis à vis de composés irritants éventuellement présents dans les compositions.

L'invention a également pour objet un procédé de traitement cosmétique pour prévenir les effets du stress oxydatif, caractérisé par le fait qu'il consiste à appliquer sur la peau une composition cosmétique comprenant une quantité efficace d'acide γ-linolénique, à laisser celle-ci en contact avec la peau, et éventuellement à rincer.

L'invention a également pour objet un procédé de traitement cosmétique pour prévenir les effets du vieillissement cutané, caractérisé par le fait qu'il consiste à appliquer sur la peau une composition cosmétique comprenant une quantité efficace d'acide γ-linolénique, à laisser celle-ci en contact avec la peau, et éventuellement à rincer.

Ce traitement cosmétique est appliqué de manière préventive.

Les exemples et compositions suivants illustrent l'invention sans la limiter aucunement. Dans les compositions les proportions indiquées sont des pourcentages en poids sauf indications contraires.

### Exemple 1 :

Mesure de l'effet protecteur de l'acide γ-linolénique sur des fibroblastes dermiques humains normaux soumis à un stress oxydatif:

Des cultures de fibroblastes dermiques humains normaux sont réalisées selon la méthode décrite par Gilchrest B. A. dans J. Gerontol., vol.35, pp. 537-541, 1980.

A J(0) on ensemence des plaques multipuits de type COSTAR à raison de 50000 cellules par puits. Les cellules sont cultivées dans du milieu MEM Eagle (GIBCO) contenant 10% de sérum de veau foetal, 2 mM de L-glutamine, 1% acides aminés non essentiels, 1 mM pyruvate de sodium et 1% d'un mélange antibiotiques/antimicotiques vendu par la société GIBCO.

A J(+1) le milieu de culture est remplacé par du milieu MEM sans sérum de veau foetal contenant ou ne contenant pas (témoin) la substance à tester.

### Traitement

Des solutions d'acides gras essentiels sont préparés dans des solutions à 0.1 % d'albumine humaine.
Les solutions mères sont à 2. 10⁻³ M , la dilution minimale est à 10⁻⁴ M dans le milieu de culture sans sérum de veau foetal.
L'addition de vitamine E à 30 µM final permet d'éviter les problèmes d'autooxydation des acides gras.
Les cellules sont traitées 3 jours sans changement de milieu.

Un stress oxydatif est induit à J(+4).
Pour cela, le milieu de culture est retiré et les plaques sont rincées avec du tampon phosphate salin (PBS).
50% des puits sont traités par une solution de terbutyl hydroperoxyde (TBH) à 10⁻⁴ M, les 2 autres puits étant laissés en PBS et l'incubation se poursuit pendant une heure dans un incubateur à 37°C en atmosphère contenant 5% de CO₂.

### Détermination de la viabilité cellulaire

Au bout d'une heure, les plaques sont rincées 3 fois au PBS pour enlever toute trace de TBH.
Une solution mère de rouge neutre à 4 mg/l est diluée au 1/40 dans un milieu minimum (MEM de marque GIBCO). La solution diluée est placée 1h au bain marie à 37°C pour une bonne dissolution puis filtrée sur 0.22 µm pour éliminer les cristaux résiduels.
La solution ainsi préparée est ajoutée sur les cellules et les plaques sont mises à l'incubateur à 37°C en atmosphère contenant 5% de CO₂ pour 3 h.
Après incubation les plaques sont vidées de leur milieu et rincées au PBS. Une solution à 0.4% de formaldéhyde et 1% de chlorure de calcium dans de l'eau est ajoutée dans chaque puits pour une durée de 1 minute.
On remplace alors cette solution par une solution à 49% d'éthanol pur dans l'eau et 1% d'acide acétique glacial.
On laisse la coloration se développer 30 mn à température ambiante et on lit les plaques au spectrophotomètre à 540 nm.

### Calcul du % de protection

Pour chaque condition (traité ou témoin), le calcul est effectué de la manière suivante :

Proportion de cellules stressées : rapport de la moyenne des densités optiques mesurées pour les puits traités sur la moyenne des densités optiques mesurées pour les puits non traités.

Pourcentage de résistance : Proportion de cellules stressées dans l'essai sur proportion de cellules stressées dans le témoin x 100

Par ce calcul, le témoin est considéré à 100 % de protection contre le stress oxydatif et les produits présentent un effet protecteur supérieur ou inférieur au témoin.

### Résultats

| a. Comparaison de l'activité de divers acides gras essentiels | |
|---|---|
| Type de traitement | Pourcentage de résistance |
| Témoin* | 100 |
| Acide Arachidonique 10 µM | 95 |
| Acide Linoléique 10 µM | 100 |
| Acide γ-Linolénique 10 µM | 124 |

| | |
|---|---|
| *sans acide gras essentiel. | |

Conclusion : Seul l'Acide γ-Linolénique dans les conditions de l'essai augmente la résistance au stress oxydatif.

| b. Effet dose de l'acide γ-linolénique : | |
|---|---|
| Doses d'acide γ-linolénique en µM | Pourcentage de résistance |
| Témoin * | 100 |
| 0,01 | 123 |
| 0,1 | 130 |
| 1 | 119 |
| 10 | 121 |
| 100 | 95 |

| | |
|---|---|
| *sans acide gras essentiel. | |

Conclusion : l'Acide γ-Linolénique présente un effet maximum à 0,1µM.

| sc. Essais comparatif avec des agents connus pour leur propriétés de protecteurs de l'oxydation. | |
|---|---|
| Type de traitement | Pourcentage de résistance |
| Acide γ-Linolénique 0,1µM | 112 |
| Témoin* | 100 |
| N-Acétyl cystéine 10 µM | 94 |
| N-Acétyl cystéine 100 µM | 103 |
| N-Acétyl cystéine 1 mM | 107 |
| Glutathion 10 µM | 95 |
| Glutathion 100 µM | 93 |
| Glutathion 1 mM | 100 |
| Vitamine E 10 µM | 112 |
| Vitamine E 100 µM | 107 |
| Vitamine E1 mM | 111 |
| Pyrrolidine dithiocarbamate 20µM | 97 |
| Pyrrolidine dithiocarbamate 100 µM | 112 |

| | |
|---|---|
| *sans acide gras essentiel. | |

Conclusion : L'acide γ-Linolénique présente un effet protecteur du stress oxydatif comparable à celui de molécules reconnues comme anti-oxydants de référence.

Exemple 2 : Exemples de formulations illustrant les compositions selon l'invention. Ces compositions ont été obtenues par simple mélange des différents composants.

| Composition 1 : Crème de nuit : | |
|---|---|
| Acide γ-linolénique | 5,0% |
| Transcutanol | 0,1% |
| Propylène glycol | 0,5% |
| Alcool | 10,0% |
| Eau | qsp 100% |

Cette composition est appliquée quotidiennement le soir sur la peau afin d'obtenir un effet préventif du stress oxydatif induit par les agressions subies dans la journée par la peau.

### Composition 2 : Patch antiride :

Le patch auto-adhésif contient 1% d'acide γ-linolénique immobilisé.
Le patch est appliqué quotidiennement le soir sur une zone ridée.

## Revendications

1. Utilisation à titre de principe actif dans une composition cosmétique d'une quantité efficace d'acide γ-linolénique, ce composé ou la composition étant destiné à prévenir les effets du stress oxydatif sur la peau.

2. Utilisation selon la revendication 1, caractérisé en ce que le composé ou la composition est destiné à prévenir le vieillissement cutané.

3. Utilisation selon l'une quelconque des revendications précédentes, caractérisée par le fait que l'acide γ-linolénique est en une proportion comprise entre 0,05% et 10% en poids par rapport au poids de la composition.

4. Utilisation selon la revendication précédente, caractérisée par le fait que l'acide γ-linolénique est en une proportion comprise entre 1% et 5% en poids par rapport au poids de la composition.

5. Procédé de traitement cosmétique pour prévenir les effets du stress oxydatif, caractérisé par le fait que l'on applique sur la peau une composition cosmétique telle que définie dans les revendications 1 à 4.

6. Procédé de traitement cosmétique selon la revendication 5, pour prévenir le vieillissement cutané.

7. Composition cosmétique comprenant dans un milieu physiologiquement acceptable de l'acide γ-linolénique à titre d'actif, destinée à prévenir les effets du stress oxydatif sur la peau.

8. Composition cosmétique selon la revendication 7, destinée à prévenir le vieillissement cutané.
